# EUROPEAN PATENT SPECIFICATION

(11) **EP 2 471 496 B1**
(45) Date of publication and mention of the grant of the patent: **24.04.2013**
(21) Application number: 12000014.6
(22) Date of filing: 02.01.2012
(51) Int. Cl.: A61F 2/64

(54) **Prosthetic knee used preferably for sports**
Knieprothese, die vorzugsweise für Sport verwendet wird
Prothèse de genou utilisé de préférence pour le sport

(30) Priority: 03.01.2011 SI 201100001
(43) Date of publication of application: 04.07.2012
(73) Proprietor: ART-LEG-d.o.o., 3310 Zalec (SI)
(72) Inventor: Zmago, Vidrih, 8000 Nova Mesto (SI)
(74) Representative: Marn, Jure

(56) References cited:
- GB-A- 2 467 201
- US-A- 5 704 946
- US-B1- 7 044 983
- US-B2- 7 785 373

## Description

### Technical problem

Currently known prosthetic knees enable customary walk, however, they do not enable sporting positions whereby person needs to be in squatting (tucked) position. The technical problem therefore is to provide for such prosthetic knee which can be used for downhill tuck or similar position of the user.

In addition, technical problem is also to enable gradual return of user position into the basic position and to reduce necessary muscle power for moving from one position into another.

### State of the Art

State of the Art features knees with pivot points into intersection of femur and tibia substitute. Such knees enable walking of the user.

The document US-B-7,785,373 is regarded as the closest prior art, wherein a prosthetic knee is disclosed, comprising an upper and lower load bearing element, a lever pivotally coupled to the upper load bearing element and a spring and schock absorber pivotally coupled to the upper and lower bearing element. This prosthetic knee has fixed frame and enables movement in one axis only which means that during the bending of the knee the center of gravity of the user is moved backwards which results in loss of ideal position of center of gravity and consequently low control over movement, worse ground contact and insecurity.

State of the Art does not feature prosthetic knees enabling squatting or tuck position known for example at skiing or other similar sports with appropriate muscle and balance control.

### Description of new invention

Prosthetic knee solves above referenced technical problem by multipoint linkage of tibia substitute, and further, using spring and gas shock absorber.

The prosthetic knee according to this invention is preferably earmarked for sporting activities following below knee or above knee amputation.

Due to unique positioning of knee axes of this invention (the knee unit is multi-axes) the same during transfer of weight onto the prosthetic knee moves both down and forward (simulation of connected effect of ankle and knee using healthy leg) which consequently means that person whose limb was amputated still maintains ideal position of center of gravity of body during sporting activity and therefore maintains appropriate control of skis or other sporting equipment.

The prosthetic knee in one of preferred embodiments for its operation uses also gas shock absorber (called also pneumatic spring), and spring which substitutes the muscle quadriceps extensor *(quadriceps femuris)* (the knee with downward pressure saves energy and releases it when the user needs it).

The choice of said gas shock absorber or other element with equal or similar effect for damping sudden movements (pneumatic spring, gas spring, etc., each of which is encompassed for purposes of this application under common name "gas shock absorber") and spring depends on sporting activity exercised by the user, his weight, muscle power, and knowledge of sporting activity performed.

The user with movement of prosthetic knee downward and consequently forward first needs to overcome resistance of gas shock absorber (in this case the initial force is chosen taking into consideration the user weight and muscle power and is constant throughout the movement) while at the same time with movement downward the force within the mechanical spring (which is also used according to user's abilities) (force of mechanical spring in the beginning of the movement does not exist as the spring is not pre-loaded, however, it increases through the movements). With preload of the mechanical spring the initial total force increases (it is therefore comprised of constant force of gas shock absorber and force of pre-loaded mechanical spring) which needs to be overcome to even bend the knee. In such a way the user can adapt - increase or decrease the force needed to bend the knee.

The prosthetic knee is in its embodiment resistant to salt and fresh water, and can be adapted to prosthesis using existing standard prosthetic pyramid adapters.

Below the invention is described using the figures whereas the figures show part of patent application.

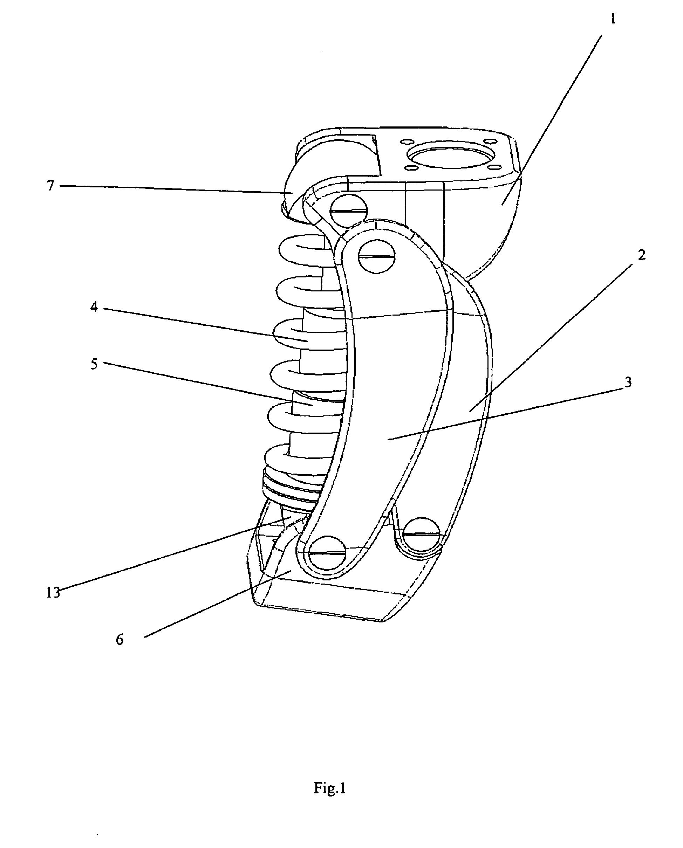

Figure 1 shows invention according to embodiment as follows: upper load bearing element 1, inner lever 2, outer lever 3, spring 4, gas shock absorber 5, lower load bearing element 6, upper bracket for gas shock absorber and spring 7, lower bracket for gas shock absorber and spring 13.

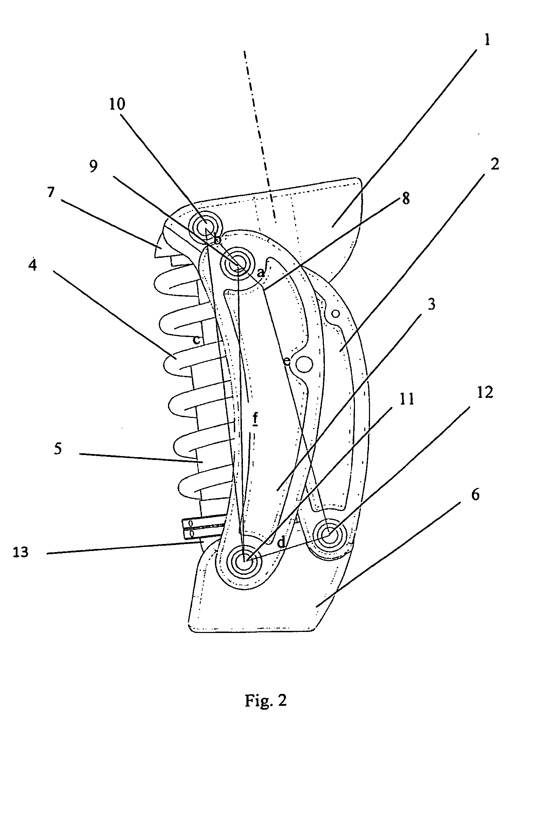

Figure 2 shows invention in upper extended position as follows: upper load bearing element 1, inner lever 2, outer lever 3, spring 4, gas shock absorber 5, lower load bearing element 6, first pivot point 8 between upper load bearing element 1 and inner lever 2, second pivot point 9 between upper load bearing element 1 and outer lever 3, third pivot point 10 between upper load bearing element 1 and upper bracket for gas shock absorber and spring 7, fourth pivot point 11 between outer lever 3, lower load bearing element 6 and lower bracket for gas shock absorber and spring 13, fifth pivot point 12 between inner lever 2 and lower load bearing element 6, connection "a" between first and second pivot points 8 and 9, connection "b" between pivot second and third points 9 and 10, connection "c" between third and fourth pivot points 10 and 11, connection "d" between fourth and fifth pivot points 11 and 12, connection "e" between fifth and first pivot points 12 and 8. There is also connection "f" between fourth and second pivot points 11 and 9.
Figure 3 shows invention in the first of three intermediate positions between upper extended position and lower flexed position.
Figure 4 shows invention in the second of three intermediate positions between upper extended position and lower flexed position.
Figure 5 shows invention in the third of of three intermediate positions between upper extended position and lower flexed position.
Figure 6 shows invention in lower flexed position.

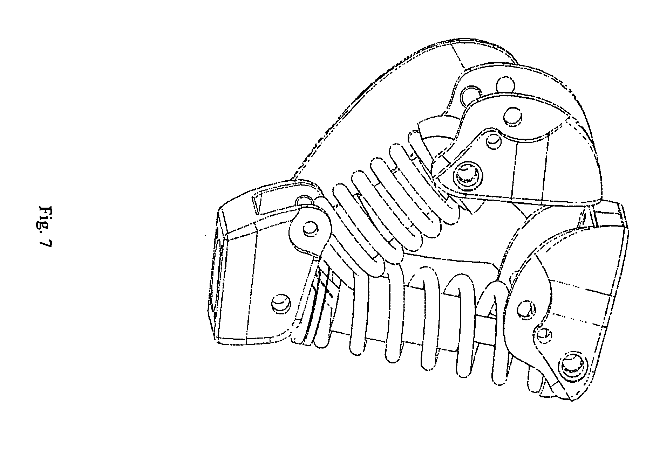

Figure 7 shows invention simultaneously in upper extended and lower flexed positions, however with parts removed (one of two inner and outer levers) said parts disturbing the view.

The invention in its embodiment comprises of upper load bearing element 1, inner lever 2, outer lever 3, spring 4, gas shock absorber 5, lower load bearing element 6, upper bracket for gas shock absorber and spring 7, lower bracket for gas shock absorber and spring 13. The upper load bearing element 1, the inner lever 2, the outer lever 3, and the lower load bearing element 6 are interconnected into kinematic structure comprising first pivot point 8 between upper load bearing element 1 and inner lever 2, second pivot point 9 between upper load bearing element 1 and outer lever 3, third pivot point 10 between upper load bearing element 1 and upper bracket for gas shock absorber and spring 7, fourth pivot point 11 between outer lever 3, lower load bearing element 6 and lower bracket for gas shock absorber and spring 13, and fifth pivot point 12 between inner lever 2 and lower load bearing element 6.

The invention formed in such a way comprises kinematic mechanism comprising connection "a" between first and second pivot points 8 and 9, connection "b" between second and third pivot points 9 and 10, connection "c" between third and fourth pivot points 10 and 11, connection "d" between fourth and fifth pivot points 11 and 12, connection "e" between pivot points 12 and 8. On figures presented herein said connections are seen as lines between appropriate pivot points however these lines in fact do not exist and are only shown for easier explanation of the invention.

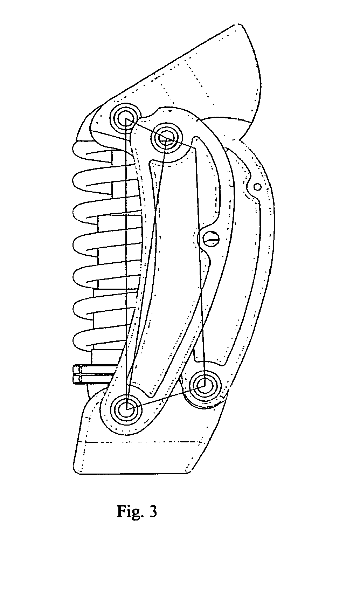

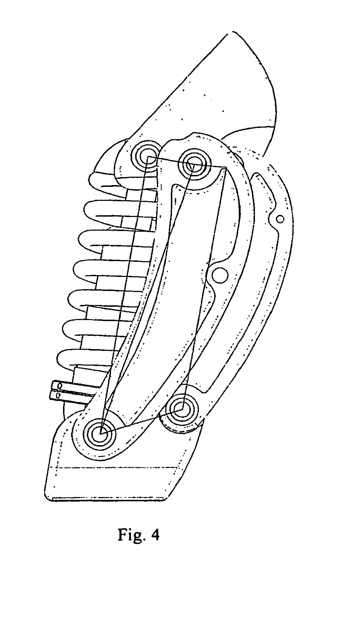

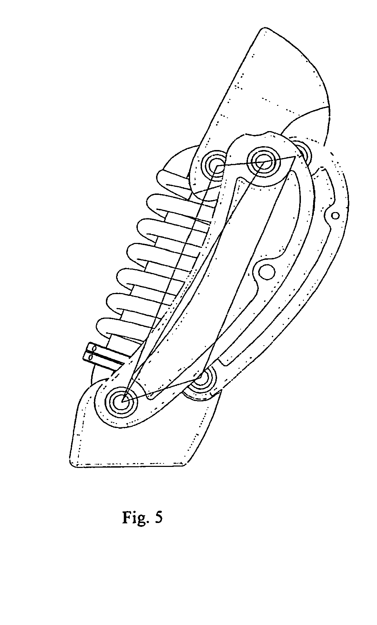

The depiction of action of this kinematic mechanism is shown in figures 2 to 6.

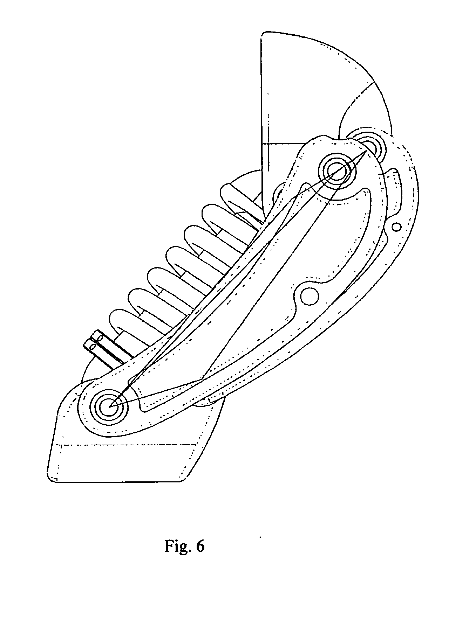

It is seen that during the movement from the upper extended position (figure 2) into lower flexed position (figure 6) the kinematic mechanism as defined by connections a through e moves in such a way to simulate interposition of the bones during moving into tuck position needed for particular sporting activities (included and not limited to alpine skiing, cross-country skiing, telemark skiing, snowboarding, wakeboarding, surfing, roller skating, roller boarding, water skiing, diving, fly-fishing, mountaineering or alpinism, skating, swimming, sledding, climbing). At moving the distances as defined by connections a, b, and d through f remain unchanged (are conserved), only angle between these connections is changing. The distance as defined by connection c is changing during said movement (is not conserved).

In particular embodiment the angle between connections a (between first and second pivot points 8 and 9) and b (between second and third pivot points 9 and 10) does not change, and this solution is even closer to moving as observed during moving of healthy people who do not need this invention.

During moving downward the user is by virtue of his or her weight compressing the spring 4 and the gas shock absorber 5. The spring 4 is conserving the energy while the gas shock absorber 5 provides for steady (smooth) compression similar to actions of muscles during moving into tuck position of healthy people.

During rising the spring 4 is extending and returning saved energy therefore helping the user during these movements while the gas shock absorber 5 again provides for steady (smooth) movements to prevent jumpy rising.

The use of this invention is also possible and also meaningful for those who do not need the prosthetic devices, namely for strengthening movements of lower extremities, for special needs (workers, soldiers etc.) with slight changes of ends and joints.

## Claims

1. The prosthetic knee for sporting use **characterized in that** it comprises upper load bearing element (1), inner lever (2), outer lever (3), spring (4), gas shock absorber (5), lower load bearing element (6), first pivot point (8) between upper load bearing element (1) and inner lever (2), second pivot point (9) between upper load bearing element (1) and outer lever (3), third pivot point (10) between upper load bearing element (1) and upper bracket for gas shock absorber and spring (7), fourth pivot point (11) between outer lever (3), lower load bearing element (6) and lower bracket for gas shock absorber and spring (13), and fifth pivot point (12) between inner lever (2) and lower load bearing element (6).

2. Prosthetic knee according to claim 1 **characterized in that** during the movements the distances are conserved namely the distances comprising the connection (a) between first and second pivot points (8) and (9), connection (b) between second and third pivot points (9) and (10), connection (d) between fourth and fifth pivot points (11) and (12), connection (e) between fifth and first pivot points (12) and (8), and connection (f) between fourth and second pivot points (11) and (9).

3. The prosthetic knee according to any of the preceding claims **characterized in that** during movement the distance as defined by connection (c) between third and fourth pivot points (10) and (11) is changing and is therefore not conserved.

4. The prosthetic knee according to any of the preceding claims **characterized in that** during the movement the angle between connection (a) between first and second pivot points (8) and (9), and connection (b) between second and third pivot points (9) and (10) is conserved.

## Patentansprüche

1. Prothetisches Knie für die Verwendung im Sport ist charakterisiert dadurch, dass es aufgebaut ist aus dem oberen Tragelement (1), dem inneren Hebel (2), dem äußeren Hebel (3), einer Feder (4), dem Gasdruckstoßdämpfer (5), dem unteren Tragelement (6), dem ersten Drehpunkt (8) zwischen dem oberen (1) und dem inneren Tragelement (2), aus dem zweiten Drehpunkt (9) zwischen dem oberen Tragelement (1) und dem äußeren Hebel (3), aus dem dritten Drehpunkt (10) zwischen dem oberen Tragelement (1) und dem oberen Träger für den Gasdruckstoßdämpfer und die Feder (7), aus dem vierten Drehpunkt (11) zwischen dem äußeren Hebel (3), dem unteren Tragelement (6) und dem unteren Träger für den Gasdruckstoßdämpfer und die Feder (13) und aus dem fünften Drehpunkt (12) zwischen dem äußeren Hebel (2) und dem unteren Tragelement (6).

2. Prothetisches Knie aus dem Anspruch 1 ist charakterisiert dadurch, dass während des Bewegens die Distanzen erhalten bleiben, und zwar weil diese Distanzen aufgebaut werden aus der Verbindung (a) zwischen dem ersten und dem zweiten Drehpunkt (8) und (9), der Verbindung (b) zwischen dem zweiten und dem dritten Drehpunkt (9) und (10), aus der Verbindung (d) zwischen dem vierten und dem fünften Drehpunkt (11) und (12) und aus der Verbindung (e) zwischen dem fünften und dem sechsten Drehpunkt (12) und (8) und aus der Verbindung (f) zwischen dem vierten und dem zweiten Drehpunkt (11) und (9).

3. Prothetisches Knie aus irgendeinem der vorherigen Ansprüche ist charakterisiert dadurch, dass während des Bewegens die Distanz, die definiert wird mit der Verbindung (c) zwischen dem dritten und dem vierten Drehpunkt (10) und (11) sich verändert und demnach nicht erhalten bleibt.

4. Prothetisches Knie aus irgendeinem der vorherigen Ansprüche ist charakterisiert dadurch, dass während des Bewegens der Winkel zwischen der Verbindung (a) zwischen dem ersten und dem zweiten Drehpunkt (8) und (9) und zwischen der Verbindung (b) zwischen dem ersten und dem dritten Drehpunkt (9) und (10) erhalten bleibt.

## Revendications

1. La prothèse de genou à usage sportif, se **caractérise par le fait qu'**elle comporte un élément porteur supérieur (1), un levier interne (2), un levier externe (3), un ressort (4), un amortisseur à gaz (5), un élément porteur inférieur (6), un premier point pivot (8) situé entre l'élément porteur supérieur (1) et le levier intérieur (2), un second point pivot situé entre l'élément porteur supérieur (1) et le levier externe (3), un troisième point pivot situé entre l'élément porteur supérieur (1) et la patte de fixation supérieure prévue pour l'amortisseur à gaz et le ressort (7), un quatrième point pivot (11) situé entre le levier externe (3), l'élément porteur inférieur (6) et la patte de fixation inférieur prévue pour l'amortisseur à gaz et le ressort (13), et un cinquième point pivot (12) situé entre le levier interne (2) et l'élément porteur inférieur (6).

2. Conformément à la revendication 1, la prothèse de genou se **caractérise par le fait que** lors des mouvements, les distances sont conservées à savoir les distances prévues pour l'articulation (a) entre le premier (8) et le deuxième point pivot (9), l'articulation (b) entre le deuxième (9) et le troisième point pivot (10), l'articulation (d) entre le quatrième (11) et le cinquième point pivot (12), l'articulation (e) entre le cinquième (12) et le premier point pivot (8), et l'articulation (f) entre le quatrième (11) et le deuxième point pivot (9).

3. Conformément aux revendications précédentes, la prothèse de genou se **caractérise par le fait que** lors du mouvement, la distance définie par l'articulation (c) entre le troisième (10) et le quatrième point pivot (11), change et n'est donc pas conservée.

4. Conformément aux revendications précédentes, la prothèse de genou se **caractérise par le fait que** lors du mouvement, l'angle entre l'articulation (a) entre le premier (8) et le deuxième point pivot (9), et l'articulation entre le deuxième (9) et le troisième (10) point pivot, est conservé.
